# EUROPEAN PATENT APPLICATION

(11) **EP 4 836 649 A1**
(43) Date of publication of application: **04.11.2026**
(21) Application number: 24913045.1
(22) Date of filing: 26.12.2024
(51) Int. Cl.: H10K 30/50, C07C 211/31, C07C 211/61, H10K 30/40, H10K 30/60, H10K 30/88, H10K 85/50

(54) **PHOTOELECTRIC CONVERSION ELEMENT FOR PEROVSKITE SOLAR CELL**

(30) Priority: 27.12.2023 JP 2023221907
(71) Applicant: Japan Science and Technology Agency, Kawaguchi-shi, Saitama 332-0012 (JP)
(72) Inventor: FUKUSHIMA, Takanori, Tokyo 152-8550 (JP); FUKUI, Tomoya, Tokyo 152-8550 (JP); SAEKI, Akinori, Suita-shi, Osaka 565-0871 (JP)
(74) Representative: Wächtershäuser & Hartz Patentanwaltspartnerschaft mbB
(86) International application number: PCT/JP2024/046251
(87) International publication number: WO 2025/143159

(57) **Abstract**

An object of the present invention is to provide a passivating agent capable of further stabilizing a perovskite type compound, and a perovskite-based photoelectric conversion element and a perovskite solar cell using the same. The present invention relates to a passivating agent for a perovskite type compound containing a tridentate cationic compound having a triptycene skeleton, and a perovskite-based photoelectric conversion element and a perovskite solar cell containing a perovskite type compound having been passivated using the passivating agent.

## Description

### Technical Field

The present invention relates to a photoelectric conversion element for a perovskite solar cell, and a passivating agent used for the photoelectric conversion element.

### Background Art

Solar cells which use compounds having a perovskite type crystal structure (perovskite type compounds) represented by ABX₃ (wherein A is a monovalent cation such as ammonium, B is a divalent metal cation such as Pb²⁺, and X is a halide ion) as a light-absorbing material are currently under development as next-generation solar cells because such solar cells have high photoelectric conversion efficiency, and can be formed into flexible, lightweight thin films by employing printing and film-deposition technologies.

Furthermore, perovskite solar cells with even higher photoelectric conversion efficiencies are being developed by replacing the monovalent cations (A) or divalent metal cations (B) with alternative materials.

The crystal surface of a perovskite type compound has a problem in stability that it reacts easily with oxygen and moisture in air. To resolve this instability, a passivating agent for passivating a perovskite type compound is under development. As the passivating agent, phenylethylammonium, alkylammonium iodides, combinations of a halogen-bond donor with a sulfur-containing compound, and metal oxides such as Al₂O₃ and TiO₂, and the like have been reported (Patent Literatures 1 to 3, and Non Patent Literature 1).

### Citation List

### Patent Literature

Patent Literature 1: WO2015/092397
Patent Literature 2: WO2018/007586
Patent Literature 3: WO2018/137048

### Non Patent Literature

Non Patent Literature 1: J. Huang et al., Adv. Mater. 2020, 32, 2000995

### Summary of Invention

### Technical Problem

However, the stabilization of perovskite type compounds for perovskite solar cells using conventional passivating agents is still insufficient, and further improvement in photoelectric conversion efficiency is desired.

Accordingly, an object of the present invention is to provide a passivating agent capable of further stabilizing a perovskite type compound, and a perovskite-based photoelectric conversion element and a perovskite solar cell using the same.

### Solution to Problem

Accordingly, the present inventors have focused on a triptycene compound having three functional groups, and have synthesized a compound in which three cations are introduced into the triptycene compound to coat the resultant compound on the surface of a perovskite type compound, and found that the perovskite type compound is stabilized and the photoelectric conversion efficiency of a photoelectric conversion element is significantly improved, and thus the present invention has been accomplished.

Specifically, the present invention provides the following inventions [1] to [12]:
[1] A passivating agent for a perovskite type compound, comprising a tridentate cationic compound having a triptycene skeleton.
[2] The passivating agent according to [1], wherein the tridentate cationic compound having a triptycene skeleton is a triptycene compound having three ammonium groups.
[3] The passivating agent according to [1] or [2], wherein the tridentate cationic compound having a triptycene skeleton is a compound of the following formula (1): 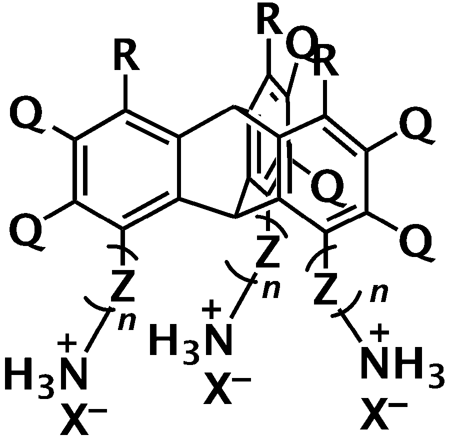
   wherein R represents a hydrogen atom, a hydroxy group, an alkoxy group, a halogen atom, a cyano group, a carboxy group, or an alkoxycarbonyl group,
   Q represents a hydrogen atom or a halogen atom,
   Z represents an optionally substituted alkylene group or oxyalkylene group, X represents a halogen, and
   n represents an integer of 0 or 1.
[4] The passivating agent according to [3], wherein the Z is an alkylene group or an oxyalkylene group.
[5] The passivating agent according to [3] or [4], wherein the Z is an alkylene group having 1 to 6 carbon atoms.
[6] The passivating agent according to any one of [3] to [5], wherein the Z is a methylene group or an ethylene group.
[7] The passivating agent according to any one of [3] to [6], wherein the n is 1.
[8] The passivating agent according to any one of [3] to [7], wherein the R is a hydrogen atom.
[9] A perovskite type compound-containing photoelectric conversion layer, comprising the passivating agent according to any one of [1] to [8] on a surface of a perovskite type compound.
[10] A perovskite-based photoelectric conversion element comprising at least a first electrode, a hole transport layer, a perovskite type compound-containing photoelectric conversion layer, an electron transport layer, and a second electrode,
   wherein the perovskite type compound-containing photoelectric conversion layer is a photoelectric conversion layer having, on a surface of a perovskite type compound, the passivating agent according to any one of [1] to [8].
[11] A solar cell comprising the photoelectric conversion element according to [10].
[12] An optical sensor comprising the photoelectric conversion element according to [10].

### Advantageous Effects of Invention

A perovskite type compound surface-treated with a passivating agent of the present invention is stably passivated, and a photoelectric conversion element containing the surface-treated perovskite type compound as a photoelectric conversion layer thereby has significantly improved photoelectric conversion efficiency. Therefore, a perovskite solar cell equipped with this photoelectric conversion element is useful as a next-generation solar cell with enhanced photoelectric conversion efficiency.

### Brief Description of Drawings

Figure 1 is a schematic cross-sectional view of a perovskite-based photoelectric conversion element of the present invention.
Figure 2 is a schematic diagram of a photoelectric conversion element for evaluation used in Examples.
Figure 3 illustrates photoelectron yield spectra and ultraviolet and visible absorption spectra of the compound A and the compound B. (a): photoelectron yield spectrum of compound A, (b): photoelectron yield spectrum of compound B, (c): ultraviolet and visible absorption spectrum of the compound A, and (d): ultraviolet and visible absorption spectrum of the compound (B). Figure 4 illustrates the intensities of photoluminescence spectra (PL) of the element 1 (the compound A) and the element 3 (control (solvent)). (a): the element 3 and (b): the element 1.
Figure 5 illustrates fluorescence decay curves (fluorescence lifetimes) of the element 1 (compound A) and the element 3 (control (solvent)). (a): the element 3 and (b): the element 1.
Figure 6 illustrates the results of contact angle measurement of the element 1 (compound A) and the element 3 (control (solvent)). (a): the element 3 and (b): the element 1.
Figure 7 illustrates current density-voltage (JV) curves of the element 1 (compound A), the element 4 (the compound A), the element 2 (the compound B), and the element 5 (the compound B). 7(a): the element 1, (b): the element 4, (c): the element 2, and (d): the element 5. Solid line: forward scan, broken line: reverse scan.
Figure 8 illustrates stability (change over time) of photoelectric conversion efficiency (PCE) of the element 4 (the compound A) and the element 5 (the compound B). 8(a): the element 4 (the compound A) and (b): the element 5 (the compound B).

### Description of Embodiments

The terms used herein are employed in their ordinary meanings as commonly used in this field, unless otherwise specified.

A solar cell is a power device which converts light energy into electrical energy by utilizing the photovoltaic effect. As a material for converting light energy into electrical energy, silicon-based materials as well as compounds such as InGaAs are used. Recently, as described above, the photoelectric conversion efficiency of a perovskite type compound has increased, and therefore the development of perovskite solar cells which use perovskite type compounds as a light absorbing material (photoelectric conversion material) has been progressing.

As described above, a perovskite type compound is a compound having a perovskite type crystal structure represented by ABX₃ (wherein A is a monovalent cation, B is a divalent metal cation, and X is a halide ion). As A, an alkylammonium cation and a phenylammonium cation are widely known, and monovalent metal ions such as Cs⁺ have also been reported. As B, divalent metal ions such as Pb²⁺, Sn²⁺, and Ge²⁺ are known.

As described above, a passivating agent is a component which inhibits the reaction of a perovskite type compound with oxygen or moisture and thereby passivates the perovskite type compound. It is known that passivation can be achieved by treating the surface of a perovskite type compound with a phenylethylammonium halide and the like, but the passivation effect thus obtained has not been sufficient.

One aspect of the present invention is a passivating agent for a perovskite type compound which contains a tridentate cationic compound having a triptycene skeleton.

The tridentate cationic compound having a triptycene skeleton is a compound in which three cationic groups are substituted on three carbon atoms constituting a compound having a triptycene skeleton. Here, the triptycene skeleton is represented by the following formula:

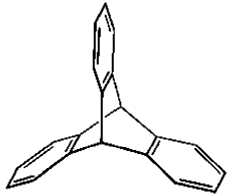

Examples of the cations substituting on the triptycene skeleton include an ammonium group (-⁺NH₃). The ammonium group may be directly substituted on a carbon atom of the triptycene skeleton, or may be substituted via an alkylene group or an oxyalkylene group.

Specific examples of the tridentate cationic compound having a triptycene skeleton include a compound in which an ammonium group, an ammoniumalkylene group, or an ammoniumalkyleneoxy group is substituted on the three carbon atoms of the triptycene skeleton. Here, the alkylene group is preferably a linear or branched alkylene group having 1 to 6 carbon atoms, more preferably an alkylene group having 1 to 3 carbon atoms, and further more preferably a methylene group or an ethylene group.

Here, counterions for the cations such as the ammonium group are preferably halide ions such as an iodide ion, a bromide ion, and a chloride ion.

The tridentate cationic compound having a triptycene skeleton is preferably a compound represented by the following formula (1): 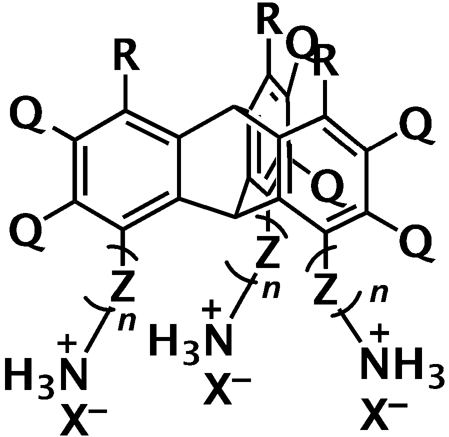
wherein R represents a hydrogen atom, a hydroxy group, an alkoxy group, a halogen atom, a cyano group, a carboxy group, or an alkoxycarbonyl group,
Q represents a hydrogen atom or a halogen atom,
Z represents an optionally substituted alkylene group or oxyalkylene group, X represents a halogen, and
n represents an integer of 0 or 1.

In the formula (1), R represents a hydrogen atom, a hydroxy group, an alkoxy group, a halogen atom, a cyano group, a carboxy group, or an alkoxycarbonyl group.

Here, the alkoxy group is preferably an alkoxy group having 1 to 6 carbon atoms, and more preferably an alkoxy group having 1 to 4 carbon atoms. Specific examples include a methoxy group, an ethoxy group, a n-propoxy group, an isopropoxy group, and a n-butoxy group.

Examples of the halogen atom include a bromine atom, a chlorine atom, a fluorine atom, and an iodine atom.

Examples of the alkoxycarbonyl group include C1-C6 alkoxycarbonyl groups, and C1-C4 alkoxycarbonyl groups are preferred. Specific examples include a methoxycarbonyl group, an ethoxycarbonyl group, and a n-propoxycarbonyl group.

R is preferably a hydrogen atom, a hydroxy group, an alkoxy group, a halogen atom, or a cyano group, more preferably a hydrogen atom, an alkoxy group, or a halogen atom, and further more preferably a hydrogen atom.

Q represents a hydrogen atom or a halogen atom. Examples of the halogen atom include a bromine atom, a chlorine atom, a fluorine atom, and an iodine atom.

Q is preferably a hydrogen atom.

Z represents an optionally substituted alkylene group or oxyalkylene group.

An example of the optionally used substituent includes a hydroxy group. The substituent is preferably an alkylene group or an oxyalkylene group.

The alkylene group is preferably a linear or branched alkylene group having 1 to 6 carbon atoms, more preferably a linear or branched alkylene group having 1 to 4 carbon atoms, further more preferably a linear or branched alkylene group having 1 to 3 carbon atoms, further more preferably a methylene or an ethylene group, and even more preferably a methylene group.

The oxyalkylene group is preferably a linear or branched oxyalkylene group having 1 to 6 carbon atoms, more preferably a linear or branched oxyalkylene group having 1 to 4 carbon atoms, further more preferably a linear or branched oxyalkylene group having 1 to 3 carbon atoms, and even more preferably an oxymethylene group or an oxyethylene group.

X represents a halogen. Examples of the halogen atom include a bromine atom, a chlorine atom, a fluorine atom, and an iodine atom.

n represents a number of 0 or 1, and is more preferably 1.

Preferred specific examples of the tridentate cationic compound having a triptycene skeleton include 1,8,13-tri(ammoniomethyl)triptycene triiodide (compound A) and 1,8,13-triammoniotriptycene triiodide (compound B).

The tridentate cationic compound having a triptycene skeleton can be produced, for example, by using trihydroxytriptycene as a starting material, and converting a hydroxy group thereof into an ammonium group, an ammoniumalkylene group, or an ammoniumalkyleneoxy group in accordance with a conventional method.

For example, 1,8,13-tri(ammoniomethyl)triptycene halide can be produced by reducing 1,8,13-tricyanotriptycene with a reducing agent such as lithium aluminum hydride to give 1,8,13-triaminotriptycene, which is then reacted with a hydrohalic acid such as hydroiodic acid.

Here, the reduction reaction may be carried out in a solvent such as tetrahydrofuran at a temperature of from room temperature to the boiling point of the solvent for 5 to 30 hours. The ammoniation reaction with a hydrohalic acid may be conducted, for example, in a solvent such as ethanol at from 0°C to room temperature for several hours.

Further, for example, 1,8,13-triammoniotriptycene trihalide can be produced by reacting 1,8,13-trihydroxytriptycene with 2-bromopropionyl amide to convert the hydroxy group into a carbamoylethoxy group, then reacting the resultant with an alkali hydroxide to give tri(2-hydroxypropionamide)triptycene through a rearrangement reaction, subsequently hydrolyzing the amide bond with an acid such as hydrochloric acid to give 1,8,13-triaminotriptycene, which is further reacted with a hydrohalic acid such as hydroiodic acid.

The reaction of 1,8,13-trihydroxytriptycene with 2-bromopropionyl amide may be carried out in a solvent such as dimethyl sulfoxide in the presence of a base such as potassium carbonate at from room temperature to 100°C for 10 to 24 hours. The subsequent rearrangement reaction may be conducted by reaction with an alkali such as potassium hydroxide in a solvent such as dimethyl sulfoxide at from 100°C to 200°C for 10 hours to 30 hours. The subsequent hydrolysis may be carried out by heating in a solvent such as ethanol using a strong acid such as concentrated hydrochloric acid. The subsequent ammoniation reaction may be conducted by using a hydrohalic acid such as hydroiodic acid, for example, in a solvent such as ethanol, at from 0°C to room temperature for several hours.

When the surface of a perovskite type compound layer is treated with a composition containing the tridentate cationic compound having a triptycene skeleton, the perovskite type compound is stably passivated, and as a result, the photoelectric conversion efficiency of a photoelectric conversion element employing the surface-treated perovskite type compound as a photoelectric conversion layer is remarkably improved. Accordingly, the tridentate cationic compound having a triptycene skeleton is useful as a passivating agent for a perovskite type compound, and a composition containing the tridentate cationic compound having a triptycene skeleton is useful as a passivating agent composition for a perovskite type compound.

Such a passivating agent or passivating agent composition may contain, in addition to the tridentate cationic compound having a triptycene skeleton, various solvents and the like.

From the viewpoint of forming a sufficient amount of a passivation layer on the surface of a perovskite type compound, the content of the tridentate cationic compound having a triptycene skeleton in the passivating agent or passivating agent composition is preferably 0.01% by mass or more and 90% by mass or less, and more preferably 0.1% by mass or more and 80% by mass or less.

As the solvent, any solvent capable of stably dissolving the tridentate cationic compound having a triptycene skeleton may be used, and examples include alcohols, and 2-propanol is preferred.

A composite layer in which a passivation layer containing the tridentate cationic compound having a triptycene skeleton is formed on one or both surfaces of a perovskite type compound layer is useful as a photoelectric conversion layer of a perovskite-based photoelectric conversion element. Thus, one embodiment of the present invention is a photoelectric conversion layer of a perovskite-based photoelectric conversion element which has the above-described passivating agent on the surface of a perovskite type compound.

From the viewpoint of achieving a sufficient passivation effect, the treatment with the passivating agent is performed so as to form a passivation layer on preferably 90% or more, more preferably 95% or more, and further more preferably 98% or more of the perovskite type compound layer.

As a treatment method with the passivating agent, any conventional film forming technique may be employed, and examples include a spin coating method, a die coating method, an inkjet coating method, and a blade coating method.

When a photoelectric conversion layer having, on one or both surfaces of the perovskite type compound layer, a passivating agent containing the tridentate cationic compound having a triptycene skeleton is used, a photoelectric conversion element aving stable and high photoelectric conversion efficiency can be obtained.

That is, another embodiment of the present invention is a perovskite-based photoelectric conversion element having at least a first electrode, a hole transport layer, a perovskite type compound-containing photoelectric conversion layer, an electron transport layer, and a second electrode, wherein the perovskite type compound-containing photoelectric conversion layer is a photoelectric conversion layer having, on a surface of the perovskite type compound, the passivating agent.

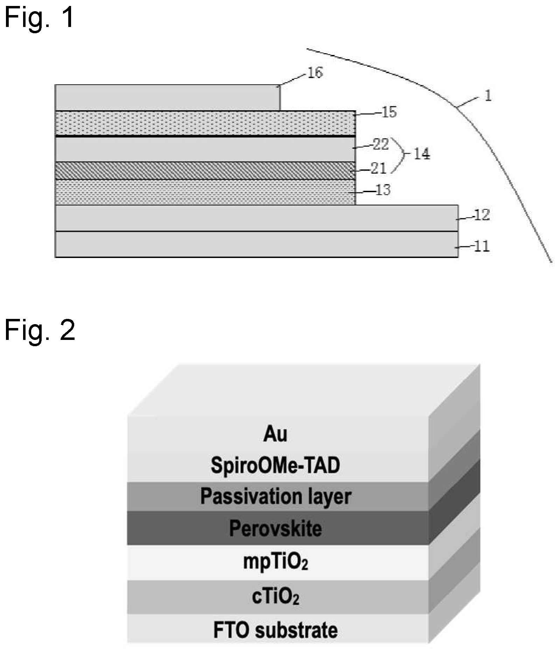

Figure 1 is a schematic cross-sectional view of the perovskite-based photoelectric conversion element of the present invention. The perovskite-based photoelectric conversion element of the present invention has a first electrode 12, an electron transport layer 13, a perovskite type compound-containing photoelectric conversion layer 14 (having a passivating agent layer 22 on the surface of a perovskite type compound 21), a hole transport layer 15, and a second electrode 16. In addition, the perovskite-based photoelectric conversion element has a substrate 11 as a case requires.

In the photoelectric conversion element 1, by applying light from outside onto the photoelectric conversion layer 14, the photoelectric conversion layer 14 absorbs the light and generates electrons and holes. The electrons generated in the photoelectric conversion layer 14 are output to the outside through the first electrode 12, while the holes are output to the outside through the second electrode 16.

The substrate 11 has a role of physically supporting the photoelectric conversion layer 14, the first electrode 12, and the second electrode 16. As the substrate 11, for example, a transparent material or a non-transparent material may be used. Examples of the transparent material include glass and transparent plastics. Examples of the non-transparent material include metals, ceramics, and opaque plastics. When a transparent material is used as the substrate 11, sunlight transmitted through the substrate can be irradiated onto the photoelectric conversion layer, and thus photoelectric conversion can be conducted.

When either or both of the first electrode 12 and the second electrode 16 have sufficient strength, the substrate 11 may be omitted. Although the substrate 11 is provided in contact with the first electrode 12 in Figure 1, the substrate 12 may instead be provided in contact with the second electrode 16.

The first electrode 12 and the second electrode 16 may be made of a conductive material. Examples of the conductive material include metals, transparent metal oxides, and carbon materials. Examples of the metals include gold, silver, copper, platinum, aluminum, titanium, nickel, tin, zinc, and chromium. Examples of the transparent metal oxides include indium-tin composite oxide, antimony-doped tin oxide, fluorine-doped tin oxide, and zinc oxide doped with boron, aluminum, gallium, or indium. Examples of the carbon materials include graphene, carbon nanotubes, and graphite.

It is desirable that either or both of the first electrode 12 and the second electrode 16 have light transmittance in the visible to near-infrared region. Even when a non-transparent material such as a metal or a carbon material is used for either the first electrode 12 or the second electrode 16, light transmittance can be imparted by providing a pattern for transmitting light. As the pattern for transmitting light, a lattice pattern, a linear pattern, and a wavy pattern can be employed.

When the first electrode 12 and the second electrode 16 are light-transmissive, their transmittance is preferably high. The transmittance is, for example, 50% or more, and is preferably 80% or more. It is also desirable that the wavelength range of the transmitted light is a broader range than the absorption wavelength range of the perovskite type compound contained in the photoelectric conversion layer 14.

When the electron transport layer 13 is provided between the photoelectric conversion layer 14 and the first electrode 12, the efficiency of extracting electrons from the first electrode 12 can be improved. The electron transport layer 13 is generally made of a semiconductor material.

Examples of the semiconductor material to be used for the electron transport layer 13 include metal oxide materials and organic n-type semiconductor materials. Examples of the metal oxide materials include titanium oxide, tin oxide, zinc oxide, and indium oxide. Examples of the organic n-type semiconductor materials include an imide compound, a quinone compound, a fullerene, and derivatives thereof.

When the hole transport layer 15 is provided between the photoelectric conversion layer 14 and the second electrode 16, the efficiency of extracting holes from the second electrode 16 can be improved. The hole transport layer 16 is generally made of a semiconductor material.

Examples of the semiconductor material to be used for the hole transport layer 16 include inorganic p-type semiconductor materials and organic p-type semiconductor materials. Examples of the inorganic p-type semiconductor materials include CuO, Cu₂O, CuSCN, molybdenum oxide, and nickel oxide. Examples of the organic p-type semiconductor materials include phenylamine and triphenylamine derivatives, which contain a tertiary amine within their skeletons, and a PEDOT compound containing a thiophene structure.

The photoelectric conversion element 1 can be produced by, for example, the following method.

First, the first electrode 12 is formed on the substrate 11. For the formation of the first electrode 12, a physical vapor deposition method or a chemical vapor deposition method can be employed. Examples of the physical vapor deposition method include a sputtering method, a resistance thermal vapor deposition method, and an electron beam evaporation method. Examples of the chemical vapor deposition method include a thermal chemical vapor deposition method, a plasma-enhanced chemical vapor deposition method, and an atomic layer deposition method.

Next, the electron transport layer 13 is formed on the first electrode 12. The electron transport layer 13 can be formed by a known method such as a spray coating method, a spin coating method, and a sputtering method.

Next, the photoelectric conversion layer 14 is formed on the electron transport layer 13. The photoelectric conversion layer 14 may be formed by applying a liquid (coating solution) containing a perovskite type compound to form a perovskite type compound layer, and then applying a solution containing the passivating agent. Examples of the coating method include a spin coating method, a die coating method, an inkjet coating method, and a blade coating method.

Subsequently, the hole transport layer 15 and further the second electrode 16 are formed on the photoelectric conversion layer 14. The hole transport layer 15 may be formed in the same manner as the electron transport layer 13, and the second electrode 16 may be formed in the same manner as the first electrode 12.

The thus obtained photoelectric conversion element has good stability and is significantly improved in photoelectric conversion efficiency, and hence can be utilized in a next-generation solar cell and an optical sensor.

Accordingly, another embodiment of the present invention is a solar cell including the aforementioned photoelectric conversion element.

Furthermore, another embodiment of the present invention is an optical sensor provided with the photoelectric conversion element.

### Examples

The present invention will now be described in more detail with reference to examples, however, the present invention is not limited to these examples.

### (Nuclear Magnetic Resonance (NMR) Measurement Method)

A solid sample to be measured was dissolved in a deuterated solvent (CD₃OD-d₄ or DMSO-d₆, manufactured by Kanto Chemical Co., Inc.) and the resulting solution was placed into an NMR tube (Wilmad NMR tubes 5 mm, manufactured by Sigma-Aldrich) to prepare a measurement sample.
[¹H-NMR Measurement Conditions]
Measurement apparatus: NMR apparatus AVANCE-400 manufactured by Bruker Corporation
Measurement frequency: 400.0 MHz
Measurement temperature: 25°C
Reference peak: CD₃OD-d₄: ¹H (δ) = 3.31 ppm, DMSO-d₆: ¹H (δ) = 2.50 ppm
[¹³C NMR Measurement Conditions]
Measurement apparatus: NMR apparatus AVANCE-500 manufactured by Bruker Corporation
Measurement frequency: 125.7 MHz
Measurement temperature: 25°C
Reference peak: CD₃OD-d₄: ¹³C (δ) = 39.52 ppm, DMSO-d₆: ¹³C (δ) = 49.00 ppm

### (Infrared Absorption Spectrum (IR) Measurement Method)

A solid sample to be measured was ground, diluted, and mixed with potassium bromide (for IR absorption measurement, manufactured by FUJIFILM Wako Pure Chemical Corporation), and the resulting mixture was then molded into a disk shape using a press molding machine to prepare a measurement sample.

### [Measurement Conditions]

Measurement apparatus: Fourier transform infrared spectrophotometer FT/IR-6600ST, manufactured by JASCO Corporation
Measurement wave number: 4000 to 400 cm⁻¹

### (Ultraviolet and Visible Absorption Spectrum (UV) Measurement Method)

A solid sample to be measured was dissolved in methanol (for spectroscopic analysis, manufactured by Tokyo Chemical Industry Co., Ltd.), and the resulting solution was placed in a quartz cell for a spectrophotometer (manufactured by GL Sciences Inc.) to prepare a measurement sample.

### [Measurement Conditions]

Measurement apparatus: Ultraviolet-visible-near-infrared spectrophotometer V-730, manufactured by JASCO Corporation
Measurement wavelength: 200 to 400 nm

### (Mass Spectrometry)

A solid sample was dissolved in methanol (manufactured by Kanto Chemical Co., Ltd.) to prepare a measurement sample.

### [Measurement Conditions]

Measurement apparatus: Time-of-flight mass spectrometer microTOF II, manufactured by Bruker Corporation
Measurement method: Atmospheric pressure chemical ionization (APCI)
Ions measured: cation

### (Photoelectron Yield Spectrum Measurement Method)

A solid sample was used as a measurement sample, and the measurement was performed under vacuum (10⁻⁴ Pa).

### [Measurement Conditions]

Measurement apparatus: Ionization energy measurement system BIP-KV202GD, manufactured by Bunkoukeiki Co., Ltd.
Measurement energy: 4 to 7 eV

### (Photoluminescence Spectrum Measurement)

### [Measurement Conditions]

Measurement apparatus: Fluorescence spectrophotometer FP-8300, manufactured by JASCO Corporation
Measurement wavelength: 700 to 870 nm

### (Fluorescence Lifetime Measurement)

### [Measurement Conditions]

Measurement apparatus: Fluoro Cube 3000U-Ultra Fast-SP spectrophotometer, manufactured by Horiba Ltd.
Measurement excitation wavelength: λₑₓ = 377 nm
Resolution: 50 ps

### (Contact Angle Measurement)

### [Measurement Conditions]

Measurement apparatus: Contact angle meter CAME1, manufactured by ASUMI GIKEN, Limited.

### (Evaluation Method for Perovskite Solar Cell)

### [Current-voltage Measurement Conditions]

Measurement apparatus: DC voltage/current source 6241A, manufactured by ADC Corporation
Light source: Solar simulator XES-301S (AM1.5G solar light at 100 mW/cm⁻²), manufactured by SANYEI CORPORATION

### [External Quantum Efficiency Measurement Conditions]

Measurement apparatus: SM-250 benchtop spectral sensitivity measurement system, manufactured by Bunkoukeiki Co., Ltd.
Source meter attached: Model 2401, manufactured by Keithley Instruments, Inc.

### (Production Example 1)

1,8,13-Tri(ammoniomethyl)triptycene triiodide (hereinafter, referred to as the compound A) was synthesized based on the following synthetic scheme. The procedure is described in order below.

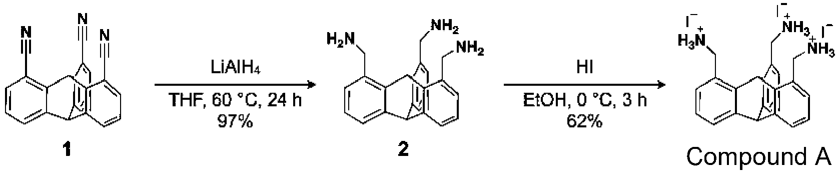

1,8,13-Tricyanotriptycene (compound 1) was synthesized in accordance with a previous report (S. Das, et al, ACS Nano 2021, 15, 11168-11179).

### (Synthesis of Compound 2)

Lithium aluminum hydride (140 mg, 3.7 mmol, manufactured by Kanto Chemical Co., Inc.) was dispersed in tetrahydrofuran (THF) (20 mL, manufactured by Kanto Chemical Co., Inc.) under a nitrogen atmosphere while maintaining the temperature at 0°C. Next, the compound 1 (200 mg, 0.61 mmol) was added to the dispersion, followed by stirring for 30 minutes. Thereafter, a saturated aqueous solution (30 mL) of potassium sodium tartrate (manufactured by FUJIFILM Wako Pure Chemical Corporation) was added dropwise to the suspension over 30 minutes. The resulting reaction mixture was extracted with dichloromethane, and the organic layer was washed three times with saturated brine. The solvent was removed from the washed organic layer under vacuum, and the resultant was vacuum dried to give a compound 2 as a white solid. The yield was 97%.

The compound 2 was subjected to NMR measurement and mass spectrometry by the methods described above. The spectral data for the compound 2 is as follows. ¹H NMR (400 MHz, DMSO-d₆, 25°C): δ (ppm) 7.30 (d, J = 6.8 Hz, 3H), 6.98-6.90 (m, 6H), 6.64 (s, 1H), 5.57 (s, 1H), 4.07 (s, 6H).

¹³C NMR (126 MHz, DMSO-d₆, 25°C): δ (ppm) 146.61, 143.44, 138.11, 125.43, 124.88, 122.51, 53, 82, 43.74, 41.29. APCI-TOF-MS (positive mode): calcd. for C₂₃H₂₃N₃: m/z = 342.1965; found: 342.2000. FT-IR (KBr) v (cm⁻¹) 3419, 3044, 2954, 2924, 1635, 1577, 1507, 1469, 1433, 1381, 1328, 1309, 1261, 1195, 1158, 1086, 1058, 1020, 996, 919, 801, 768, 723, 701.

### (Synthesis of Compound A)

Under a nitrogen atmosphere, the compound 2 (130 mg, 0.38 mmol) was dissolved in ethanol (3 mL), and the resulting solution was cooled to 0°C. To this solution, hydroiodic acid (concentration: 57% by mass, 7.2 mL, manufactured by Kanto Chemical Co., Inc.) was slowly added dropwise, followed by stirring at 0°C for 3 hours. When diethyl ether was added to the reaction solution, a yellow precipitate formed. The obtained precipitate was collected by filtration and dried under vacuum at 60°C to give a compound A as a yellow solid. The yield was 62%.

The compound A was subjected to NMR and IR measurement by the methods described above. The spectral data for the compound A is as follows.

¹H NMR (500 MHz, CD₃OD-d₄, 25°C): δ (ppm) 7.54 (d, J = 6.5 Hz, 3H), 7.22-7.17 (m, 6H), 6.56 (s, 1H), 5.76 (s, 1H), 4.78 (s, 6H).

¹³C NMR (126 MHz, CD₃OD-d₄, 25°C): δ (ppm) 148.13, 143.85, 130.04, 127.65, 126.85, 125.72, 55.763, 43.560, 41.955. FT-IR (KBr): v (cm⁻¹) 3446, 3014, 2926, 2690, 2591, 1622, 1589, 1558, 1541, 1489, 1458, 1435, 1388, 1200, 1161, 1109, 1067, 1032, 801, 770, 720.

Anal. Calcd for C₂₃H₂₆N₃I₃: C, 38.09; H, 3.61; N, 5.79; I, 52.50. Found: C, 37.94; H, 3.82; N, 5.67.

### (Production Example 2)

1,8,13-Triammoniotriptycene triiodide (hereinafter, referred to as the compound B) was synthesized based on the following synthetic scheme. The procedure is described in order below.

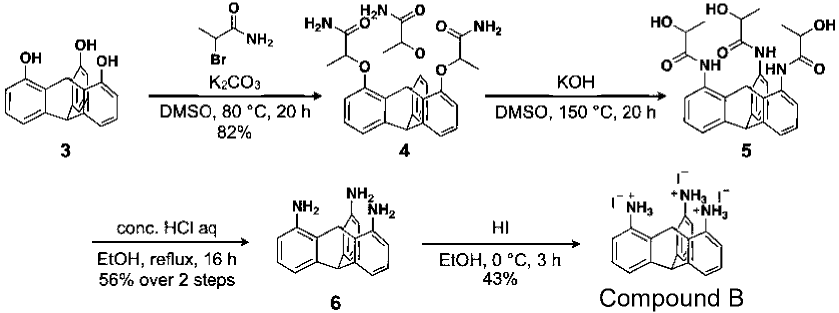

1,8,13-Trihydroxytriptycene (3) was synthesized in accordance with a previous report (N. Seiki, et al, Science 2015, 348, 1122-1126).

### (Synthesis of Compound 4)

Under a nitrogen atmosphere, the compound 3 (2.0 g, 6.6 mmol), 2-bromopropionyl amide (6.0 g, 40 mmol, manufactured by Merck), and potassium carbonate (8.2 g, 59 mmol, manufactured by FUJIFILM Wako Pure Chemical Corporation) were dissolved in dimethyl sulfoxide (DMSO) (55 mL, manufactured by Kanto Chemical Co., Inc.), and the resultant mixture was heated to 80°C, followed by stirring for 20 hours. The reaction solution was cooled to room temperature, and was then poured into water, which resulted in the formation of a white precipitate. The obtained precipitate was collected by filtration, washed with water, and dried under vacuum to give a compound 4.

The compound 4 was subjected to NMR measurement by the method described above, and the NMR spectral data for the compound 4 is as follows.

¹H NMR (400 MHz, DMSO-d₆, 25°C): δ (ppm) 7.55-7.32 (m, 6H), 7.12-7.09 (m, 3H), 6.95-6.91 (m, 3H), 6.75-6.61 (m, 4H), 6.68-5.63-5.61 (m, 1H), 4.67-4.58 (m, 3H), 1.57-1.50 (m, 9H).

### (Synthesis of Compound 6)

Under a nitrogen atmosphere, the compound 4 (3.0 g, 5.8 mmol) and potassium hydroxide (manufactured by FUJIFILM Wako Pure Chemical Corporation) (1.6 g, 29 mmol) were dissolved in DMSO (55 mL), followed by stirring at 150°C for 24 hours. The reaction solution was cooled to 25°C, dispersed in water, and then extracted with ethyl acetate. The organic layer was dried over anhydrous sodium sulfate, and the solvent was removed under vacuum. The thus obtained solid was dried under vacuum at 60°C to obtain a compound 5. The compound 5 was then dissolved in ethanol (120 mL), and to this solution, hydrochloric acid (concentration: 35% by mass, 50 mL) and water (30 mL) were added, followed by heating under reflux for 16 hours. The reaction solution was cooled to room temperature, and was then diluted with water and neutralized using a saturated aqueous sodium bicarbonate solution. The resulting aqueous layer was extracted with ethyl acetate, and the organic layer was washed with water and saturated brine. After the organic layer was dried over anhydrous sodium sulfate, the solvent was removed under vacuum. The thus obtained solid was recrystallized from ethyl acetate to give a compound 6 as a white solid at yield of 46%.

The compound 6 was subjected to NMR and IR measurement, and mass spectrometry by the methods described above, and ¹H and ¹³C NMR spectra of the compound 6 are as follows.

¹H NMR (400 MHz, DMSO-d₆, 25°C): δ (ppm) 6.62-6.57 (m, 6H), 6.26 (d, J = 7.0 Hz, 3H), 6.06 (s, 1H), 5.20 (s, 6H), 5.09 (s, 1H).

¹³C NMR (126 MHz, DMSO-d₆, 25°C): δ (ppm) 147.76, 142.73, 128.87, 125.06, 112.50, 112.01, 54.966, 34.891.

FT-IR (KBr) v (cm⁻¹) 3425, 2958, 2929, 1542, 1479, 1445, 1339, 1294, 1254, 1181, 938, 862, 795, 729, 681, 516. APCI-TOF-MS (positive mode): calcd. for C₂₀H₁₇N₃: m/z = 300.1495; found: 300.1504.

### (Synthesis of Compound B)

Under a nitrogen atmosphere, the compound 6 (100 mg, 0.33 mmol) was dissolved in ethanol (2 mL) and cooled to 0°C. To the resultant, hydroiodic acid (concentration: 57% by mass, 5 mL) was slowly added, followed by stirring at 0°C for 3 hours. Diethyl ether was added to the reaction solution, and a yellow precipitate was thereby formed. The obtained precipitate was collected by filtration and dried under vacuum at 60°C to give a compound B as a yellow solid at yield of 43%.

The compound B was subjected to NMR and IR measurement by the methods described above, and ¹H and ¹³C NMR spectra of the compound B are as follows.

¹H NMR (400 MHz, DMSO-d₆, 25°C): δ (ppm) 7.17 (d, J = 7.6 Hz, 3H), 6.97 (dd, J = 7.6 Hz, 3H), 6.76 (d, J = 7.6 Hz, 3H), 6.18 (s, 1H), 5.63 (s, 1H).

¹³C NMR (126 MHz, DMSO-d₆, 25°C): δ (ppm) 147.80, 134.23, 132.28, 127.24, 121.19, 118.52, 53.143, 37.135.

FT-IR (KBr) v (cm⁻¹) 3567, 3419, 2876, 2552, 1597, 1558, 1542, 1507, 1474, 1444, 1238, 1200, 1173, 1162, 1081, 792, 761, 722.

Anal. Calcd for C₂₀H₂₀N₃I₃: C, 35.17; H, 2.95; N, 6.15; I, 55.73. Found: C, 34.84; H, 3.11; N, 6.37.

The molecular structures of 1,8,13-tri(ammoniomethyl)triptycene triiodide (compound A) and 1,8,13-triammoniotriptycene triiodide (compound B) are as follows.

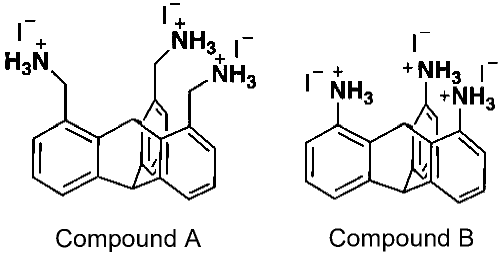

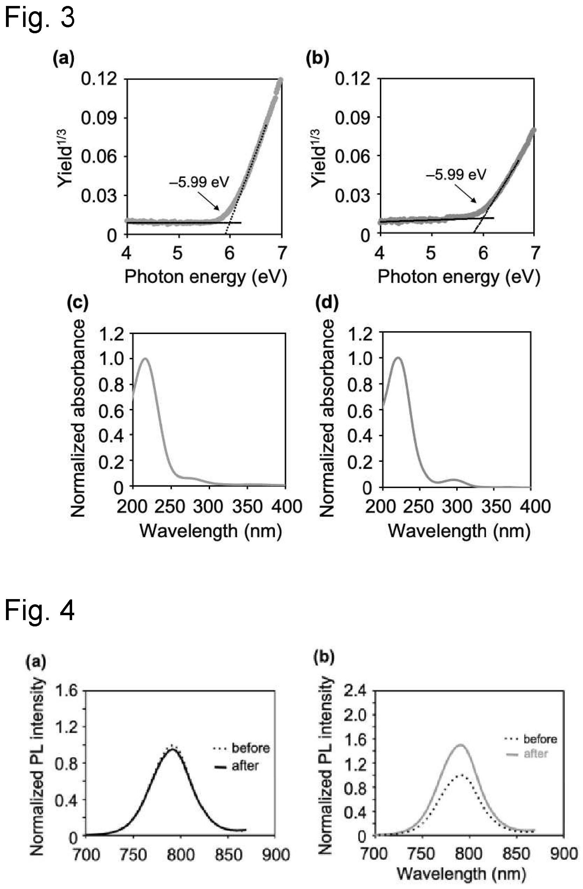

The photoelectron yield spectroscopy and the ultraviolet absorption spectroscopy were conducted under the above-described conditions. The measurement results are illustrated in Figure 3.

Based on the photoelectron yield spectroscopy and the absorption spectroscopy, deep HOMO levels (-5.99 eV) and wide band gaps (> 3.8 eV) were obtained for the compound A and the compound B. These results indicate that thin films formed using the compound A and the compound B may function as passivating agents rather than hole transport layers.

### (Production of Perovskite Solar Cell)

A FTO (fluorine-doped tin oxide) transparent conductive glass (manufactured by Sigma-Aldrich, surface resistance: 7 Ω) was used as a substrate. The FTO layer on the substrate was etched with 6M hydrochloric acid and metallic zinc (Zn, manufactured by FUJIFILM Wako Pure Chemical Corporation) using a masking tape. Next, the resultant FTO layer was washed sequentially with a detergent, acetone, isopropyl alcohol, and deionized water, and then, a compact TiO₂ layer (hereinafter referred to as the cTiO₂ layer) was deposited on the FTO layer by spray pyrolysis of an ethanol solution (1:14 v/v) of titanium diisopropoxide bis(acetylacetonate) (manufactured by Tokyo Chemical Industry Co., Ltd.) at 450°C.

A TiO₂ paste (PST-30NR-D, manufactured by GreatCell Solar Ltd.) diluted with ethanol (paste:ethanol = 1:7 w/w) was spin coated onto the cTiO₂ layer (ramp 3 s, 5,000 rpm for 15 s, ramp 2 s), followed by calcination at 500°C for 20 minutes, and thus, a mesoporous TiO₂ layer (hereinafter referred to as the mpTiO₂ layer) having a thickness of 200 nm (average particle size: 30 nm, anatase) was deposited.

Next, a solution of formamidinium hydroiodide ((NH₂)₂CHI), manufactured by Tokyo Chemical Industry Co., Ltd., hereinafter referred to as FAI), lead iodide (PbI₂, manufactured by Tokyo Chemical Industry Co., Ltd.), methylammonium bromide (CH₃NH₃Br, manufactured by Tokyo Chemical Industry Co., Ltd., hereinafter referred to as MABr), and lead bromide (PbBr₂, manufactured by Tokyo Chemical Industry Co., Ltd.) in 1.4 M N,N'-dimethylformamide (DMF, super dehydrated, manufactured by FUJIFILM Wako Pure Chemical Corporation):dimethyl sulfoxide (DMSO, super dehydrated, manufactured by FUJIFILM Wako Pure Chemical Corporation) = 4:1 (v/v) was mixed in a nitrogen-filled glovebox at a stoichiometric ratio (MABr:PbBr₂:FAI:PbI₂ = 0.13:0.13:0.87:0.87) to prepare a perovskite precursor solution.

Next, the perovskite precursor solution was spin coated onto the mp-TiO₂ layer to form a precursor layer (ramp 1 s, 1,000 rpm for 10 s, ramp 4 s, 4,500 rpm for 30 s, ramp 2 s). After 35 seconds, an antisolvent treatment was performed by slowly dropping 180 µL of anhydrous chlorobenzene 99.8% (manufactured by Sigma-Aldrich) onto the precursor layer on the rotating substrate.

By heat-treating the resulting transparent film at 100°C for 40 min, the precursor layer was converted into a perovskite compound, and a perovskite layer having a thickness of 300 nm was thereby formed.

A passivation layer was formed on the perovskite layer by spin casting, at 5000 rpm for 15 seconds, an isopropyl alcohol solution (0.5 to 3.0 mg/mL) containing the tridentate cationic compound having a triptycene skeleton (compound A or compound B).

A hole transport layer was formed on the passivation layer or on the perovskite layer by spin-coating a chlorobenzene (manufactured by Sigma-Aldrich, anhydrous, 99.8%) solution (concentration: 78.2 mg/mL) containing Spiro-OMeTAD (manufactured by Borun New Material Technology Ltd.) which was a hole transport material dissolved in acetonitrile (manufactured by Sigma-Aldrich, anhydrous, 99.8%), 2.9 vol% of 4-tert-butylpyridine (TBP, manufactured by Sigma-Aldrich, 98%) relative to the solution volume, and 52 mol% of (trifluoromethanesulfonyl)imide (LiTFSI, manufactured by Tokyo Chemical Industry Co., Ltd.) and 5.4 mol% of Co(TFSI)₂ (FK209, manufactured by Lumtec Inc.) relative to Spiro-OMeTAD, and thus, a Spiro-OMeTAD thin film having a thickness of 50 nm was formed.

Furthermore, a stripe-shaped gold electrode having a thickness of 70 nm was deposited by thermal vapor deposition in a vacuum chamber. The resultant was then stored in an oxygen-replaced desiccator in the dark for three days to promote oxygen doping into the hole transport layer, and thus, a photoelectric conversion element for evaluation was obtained (Figure 2).

### (Example 1)

### (Study Using Thin Films made of Compound A and Compound B as Passivation Layers)

A passivation layer was formed on a binary mixed perovskite (hereinafter referred to as PVK), having the composition MA_{0.13}FA_{0.87}PbI_{2.61}Br_{0.39} [MABr (or PbBr₂):FAI (or PbI₂) = 0.13:0.87] by spin coating an isopropyl alcohol (IPA) solution of the compound A. The concentration of the compound A in the IPA solution was 1.0 mg/mL. The thus obtained photoelectric conversion element is hereinafter referred to as the element 1.

### (Example 2)

A passivation layer was formed in the same manner as in Example 1 except that the compound B was used instead of the compound A. The thus obtained photoelectric conversion element is hereinafter referred to as the element 2.

### (Comparative Example 1)

To compare the effect of the passivation layer, a control sample was prepared by spin coating IPA alone onto the PVK. This sample is hereinafter referred to as the element 3.

Figure 4 illustrates the photoluminescence spectra measured in the elements 1 and 3 by the method described above. The element 3 (solvent only) exhibited substantially the same photoluminescence (PL) intensity as before forming the passivation layer (Figure 4(a)). The element 1 using the compound A, exhibited a photoluminescence (PL) intensity 1.49 times higher than that before forming the passivation layer (Figure 4(b)).

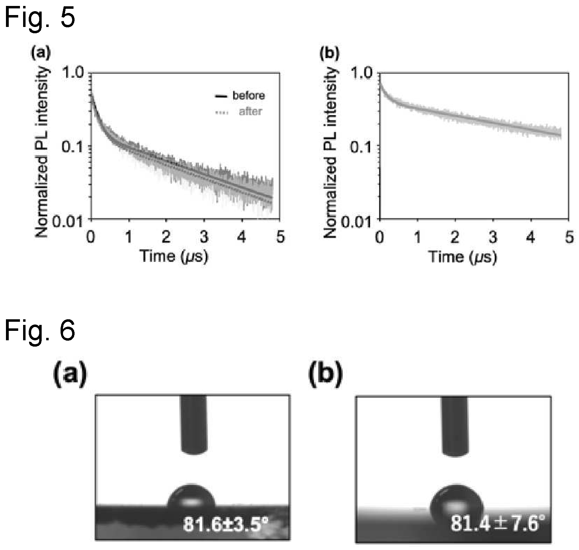

Figure 5 illustrates the fluorescence lifetimes of the elements 1 and 3 measured by the method described above. The element 1 using the compound A exhibited a longer average lifetime than the element 3 not having the passivation layer.

All results of PL decay and double exponential analyses are shown respectively in Table 1.

These results indicate that the compound A having a flexible ammoniomethyl moiety, coordinates to A-site vacancies on the PVK film surface, and hence can effectively passivate defects on the PVK interface.

**[Table 1]**

| Fluorescence (770 nm) Decay Curves of PVK Films before/after Spin Coating | | | | |
|---|---|---|---|---|
| Treatment Method ^{a)} | Concentration [mg mL⁻¹] | *τ*1 [ns] (intensity [%]) | *τ*2 [ns] (intensity [%]) | *τ*ave [ns] ^{b)} |
| | 0 (IPA) | 167.4 (75.9) | 2321.2 (24.1) | 686.4 |
| Compound A | 1.0 | 178.6 (44.1) | 4516.0 (55.9) | 2605.2 |

| | | | | |
|---|---|---|---|---|
| ^{a)} N/A: No example was conducted. IPA stands for isopropyl alcohol. ^{b)} Average lifetime **=** (*A*_{1 *τ*1}*+ A*_{2 *τ*2})( *A*₁ + *A*₂)⁻¹. | | | | |

### (Contact Angle Measurement)

The contact angles of the elements 1 and 3 were measured by the method described above. The results are illustrated in Figure 6. Figure 6(a) illustrates the result for the element 3 (solvent only), and Figure 6(b) illustrates the result for the element 1.

The element 1 using the compound A exhibited, after forming the passivation layer, a contact angle comparable to that of the element 3.

### (Example 3)

A passivation layer was formed in the same manner as in Example 1 except that the concentration of the compound A in the IPA solution was changed to 1.5 mg/mL. The thus obtained photoelectric conversion element is hereinafter referred to as the element 4.

### (Example 4)

A passivation layer was formed in the same manner as in Example 3 except that the compound B was used instead of the compound A. The thus obtained photoelectric conversion element is hereinafter referred to as the element 5.

### (Example 5)

### (Evaluation of Perovskite Solar Cell)

The current-voltage curve was measured using a light source/voltage meter unit (ADCMT Corporation, 6241A) under irradiation with AM 1.5G solar light at 100 mW cm⁻² (one sun, monitored with a calibrated standard cell, Bunko-Keiki BS-520BK) by a 300 W solar simulator (SANYEI CORPORATION, XES-301S). The size of the active area was defined by a black metal mask with a square aperture (2 × 2 mm²). The external quantum efficiency (EQE) spectrum was measured using Bunko-Keiki model SM-250KD equipped with a Keithley model 2401 source meter. The monochromatic light power was calibrated using a silicon photodiode (Bunko-Keiki model S1337-1010BQ).

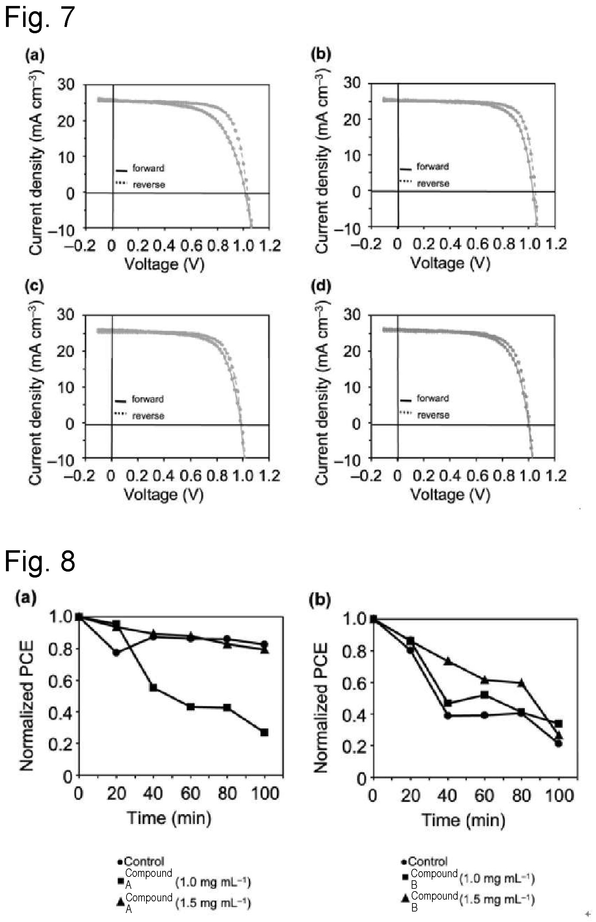

Figure 7 illustrates the current density-voltage (JV) curves of the perovskite solar cells (PSCs) [structure: fluorine-doped tin oxide (FTO)/compact TiO₂/mp-TiO₂/PVK (with/without passivation layer)/Spiro-OMeTAD/Au] (see Figure 2).

As a result, it is evident from the comparison of the power conversion efficiencies (PCEs) under the treatment of the highest performance that the PSC passivated with the compound A has a PCE of 20.25%, which is higher than those of the control (18.92%) and the PSC passivated with the compound B (18.92%). Thus, the performance is improved.

As shown in Table 2, the passivation layer prepared using the solution with a concentration of 1.5 mg/mL was the optimal. The hysteresis of the PSC passivated with the compound A was slightly larger than that of the control, and as a result, the average PCE values of these two cells were substantially the same [control: 17.38 ± 1.10%, compound B: 17.37 ± 0.99%]. The improvements in open-circuit voltage (VOC) and fill factor (FF) resulting from the passivation treatment with the compound A represent a pronounced passivation effect consistent with the PL measurements. The short-circuit current density (JSC) showed almost no change.

**[Table 2]**

| Performance Evaluation of Solar Cells Passivated with Compound A or B^{a)} | | | | | | | |
|---|---|---|---|---|---|---|---|
| Treatment ^{b)} | Concentration (mg mL⁻¹) | PCE (%) | *J*_{SC} (mA cm⁻²) | *V*_{OC} (V) | FF | HI^{c)} | # of device |
| Control Element 3 | 0 | 17.38± 1.10 (18.92) | 24.46±1.14 (25.23) | 1.001± 0.015 (1.008) | 0.709± 0.017 (0.744) | 0.03± 0.10 (0.00) | 9 |
| Element 1 | 1.0 | 17.15± 1.00 (19.21) | 24.22±1.40 (25.60) | 1.037± 0.010 (1.033) | 0.683± 0.023 (0.727) | 0.09± 0.10 (0.03) | 9 |
| Element 4 | 1.5 | 17.37± 0.99 (20.25) | 24.00±1.27 (25.23) | 1.023± 0.016 (1.049) | 0.707± 0.022 (0.765) | 0.10± 0.10 (0.10) | 10 |
| Element 2 | 1.0 | 16.58± 0.87 (18.37) | 24.92±0.84 (25.37) | 0.978± 0.017 (0.991) | 0.681± 0.022 (0.730) | 0.08± 0.08 (0.07) | 6 |
| Element 5 | 1.5 | 14.63± 1.35 (18.81) | 24.22±1.18 (25.50) | 0.956± 0.029 (0.997) | 0.632± 0.041 (0.720) | 0.07± 0.15 (0.10) | 6 |

| | | | | | | | |
|---|---|---|---|---|---|---|---|
| ^{a)} FTO/cTiO₂/mpTiO₂/PVK/(passivation)/doped SpiroOMeTAD/Au. Each value in parentheses represents the maximum value obtained in the reverse scan. ^{b)} Surface treatment with isopropyl alcohol solution. ^{c)} Hysteresis index = (PCE in reverse scan - PCE in forward scan)/(PCE in reverse scan) | | | | | | | |

Figure 8 illustrates the results of the stability evaluation of the PSCs. The elements were stored in air under conditions of a temperature of 23°C and a relative humidity of 75%.

It is evident that the elements 1 and 4 treated with the compound A as the passivation layers, have higher stability compared with their stabilities prior to the formation of the passivation layers.

The element 2 treated with the compound B has stability comparable to that before forming the passivation layer. In contrast, it is evident that the element 5 formed by increasing the concentration of the solution of the compound B during spin coating to increase the amount deposited, has improved stability.

### Reference Signs List

- 1: photoelectric conversion element
- 11: substrate
- 12: first electrode
- 13: electron transport layer
- 14: photoelectric conversion layer
- 15: hole transport layer
- 16: second electrode
- 21: perovskite type compound
- 22: passivation layer

## Claims

1. A passivating agent for a perovskite type compound, comprising a tridentate cationic compound having a triptycene skeleton.

2. The passivating agent according to claim 1, wherein the tridentate cationic compound having a triptycene skeleton is a triptycene compound having three ammonium groups.

3. The passivating agent according to claim 1, wherein the tridentate cationic compound having a triptycene skeleton is a compound of the following formula (1): 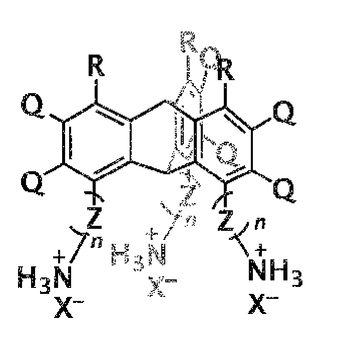
wherein R represents a hydrogen atom, a hydroxy group, an alkoxy group, a halogen atom, a cyano group, a carboxy group, or an alkoxycarbonyl group,
Q represents a hydrogen atom or a halogen atom,
Z represents an optionally substituted alkylene group or oxyalkylene group, X represents a halogen, and
n represents an integer of 0 or 1.

4. The passivating agent according to claim 3, wherein the Z is an alkylene group or an oxyalkylene group.

5. The passivating agent according to claim 3, wherein the Z is an alkylene group having 1 to 6 carbon atoms.

6. The passivating agent according to claim 3, wherein the Z is a methylene group or an ethylene group.

7. The passivating agent according to claim 3, wherein the n is 1.

8. The passivating agent according to claim 3, wherein the R is a hydrogen atom.

9. A photoelectric conversion layer for a perovskite-based photoelectric conversion element, comprising the passivating agent according to any one of claims 1 to 8 on a surface of a perovskite type compound.

10. A perovskite-based photoelectric conversion element comprising at least a first electrode, a hole transport layer, a perovskite type compound-containing photoelectric conversion layer, an electron transport layer, and a second electrode,
wherein the perovskite type compound-containing photoelectric conversion layer is a photoelectric conversion layer having, on a surface of a perovskite type compound, the passivating agent according to any one of claims 1 to 8.

11. A solar cell comprising the photoelectric conversion element according to claim 10.

12. An optical sensor comprising the photoelectric conversion element according to claim 10.
